(19) Europäisches Patentamt
European Patent Office
Office européen des brevets

(11) **EP 4 015 094 A1**

(12) **EUROPEAN PATENT APPLICATION**

(43) Date of publication:
**22.06.2022 Bulletin 2022/25**

(21) Application number: **20214450.7**

(22) Date of filing: **16.12.2020**

(51) International Patent Classification (IPC):
**B05B 17/06** (2006.01)　　　**A61M 15/00** (2006.01)
**B05B 17/00** (2006.01)

(52) Cooperative Patent Classification (CPC):
**B05B 17/0646; A61M 11/005; A61M 15/0085;
B05B 17/063;** A61M 15/0021; A61M 2016/0021;
A61M 2016/0027; A61M 2202/0468;
A61M 2205/3386; A61M 2205/50; A61M 2205/8206

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Vectura Delivery Devices Limited
Chippenham, Wiltshire SN14 6FH (GB)**

(72) Inventor: **Weiser, Yannic
Cambridge, Cambridgeshire CB40GZ (GB)**

(74) Representative: **Clarke, Christopher John
Vectura Limited
205 Cambridge Science Park
Milton Road
Cambridge CB4 0GZ (GB)**

(54) **VIBRATING MEMBRANE NEBULIZER**

(57)　The present invention provides an inhalation device comprising: an aerosol generator comprising a vibrator and a membrane; and a reservoir for liquid to be aerosolized which is in fluid communication with the membrane. A method for operating the device is also provided. Scans are performed in which an electrical parameter of the vibrator is measured as the membrane is vibrated at a plurality of frequencies. The stimulus which causes the membrane to vibrate is adjusted in response to the measured value of the electrical parameter, in order to maintain a constant value of the electrical parameter, or where a constant value of the electrical parameter cannot be maintained, to reduce the decrease in the value of the electrical parameter.

FIG. 5

EP 4 015 094 A1

## Description

### Technical Field of the Invention

The present invention relates to a vibrating membrane nebulizer, and in particular to a nebulizer which performs frequency scans in order to determine a resonant frequency and / or whether liquid is present on the membrane.

### Background to the Invention

Aerosols for medical inhalation therapy generally comprise an active ingredient dissolved or suspended in an aerosolisable liquid (often water). A homogeneous distribution of aerosol droplets with a droplet size of around 5 $\mu$m is required in order to reach deep into the lungs. Vibrating membrane nebulizers are one type of device for producing such aerosols. These devices have an aerosol generator which comprises a vibrator, such as piezoelectric element which is excited at ultrasonic frequencies in order to induce vibration; a membrane (sometimes called a mesh or aperture plate), which has a large number of holes which typically have a diameter of 1 $\mu$m to 10 $\mu$m; and a reservoir, which supplies the liquid drug formulation to the membrane.

Some vibrating membrane nebulizers have systems for monitoring the electrical parameters of the aerosol generator during use. By detecting changes in an electrical parameter (e.g. the current consumption), it is possible to infer information about mechanical changes in the aerosol generator, such when all the liquid is used up. This can be used to prevent damage to the membrane by stopping the vibration when it becomes dry. For example, WO2014/062175 and WO2015/091356 disclose nebulizers which perform scans periodically during nebulization in which the impedance of the piezoelectric element and the voltage applied to the piezoelectric element respectively are measured at a series of different frequencies. The resulting curves are analyzed to determine whether liquid is present on the membrane or not.

Moreover, in some vibrating mesh nebulizers, the resonant frequency of the aerosol generator can change as the amount of liquid within the reservoir decreases. Since the vibration frequency may depend on, or be chosen according to, the resonant frequency of the aerosol generator, it is necessary to determine the resonant frequency at intervals throughout operation of the aerosol generator. This can be done by performing a scan which spans the frequency range in which resonance occurs. A peak in the resulting spectrum occurs at the resonant frequency.

However, a disadvantage with performing such frequency scans for empty detection and/or resonant frequency measurement is that the aerosol output rate may be reduced. This is because most of the frequencies at which the membrane is vibrated during the scan are not the optimum driving frequency for aerosol generation.

### Brief Description of the Invention

The present invention addresses this problem and provides an improved way of performing such scans. Accordingly, in a first aspect, the present invention provides an inhalation device comprising:

- an aerosol generator having a vibrator and a membrane,
- a reservoir for liquid to be aerosolized which is fluidically connected to the membrane,
- a controller which is configured to (i) provide a driver signal to operate the vibrator so that the membrane vibrates and generates an aerosol, the driver signal comprising a frequency and a duty cycle; (ii) perform scans in which the membrane is vibrated at a plurality of frequencies; and (iii) measure an electrical parameter of the aerosol generator as a function of frequency during the scan;

characterized in that the controller is a closed-loop controller which is configured to adjust the duty cycle during the scan in response to the measured value of the electrical parameter in order to minimize a decrease in the value of the electrical parameter.

The invention addresses the problem of reduced aerosol output during a scan by increasing the duty cycle in order to maintain a constant value of the electrical parameter (so that the aerosol output rate is maintained), or at least to reduce the decrease in the electrical parameter or at frequencies where it cannot maintain a constant value. Thus, in contrast to the known methods which perform scans with a constant stimulus (e.g. the duty cycle) and measure the effect of varying the frequency on an electrical parameter (e.g. current, voltage or impedance), in the present invention, the scan is performed with a variable stimulus while maintaining, as far as possible, a constant value of the electrical parameter (and hence aerosol output rate) by means of closed loop feedback.

The inhalation device may comprise a battery, a transformer and a power converter. The controller may provide the driver signal to the power converter. The inhalation device may comprise a shunt resistor in series with the input of the power converter for measuring the electrical parameter. The electrical parameter may be the current drain of the power converter.

The aerosol generator may comprise a support member on which the vibrator and / or the membrane are mounted. The vibrator may be an annular piezoelectric element. The support element may be a transducer in the form of a hollow tubular body having a flange at or close to a first end onto which the piezoelectric element is attached, and a second end into or onto which the membrane is mounted. The device may comprise a filling chamber located above, and in fluid communication with, the support member, so that the filling chamber and the hollow tubular body together form the reservoir. In this type of nebulizer, the resonant frequency may depend quite strongly on the liquid level in the reservoir due to the tubular shape of the cavity. Consequently, it may be necessary to repeatedly adjust the frequency of the driver signal; the present invention prevents the repeated scans from causing a significant decrease in the aerosol output rate.

Alternatively, the support member may comprise an essentially planar annulus or disk, with the membrane and / or the vibrator may be mounted on the support member, for example on opposite sides.

The controller may be configured to determine a resonant frequency of the aerosol generator from the scan. The controller may be configured to drive the vibrator at the resonant frequency, or at a frequency that is related to the resonant frequency, such as a fixed offset from the resonant frequency, during the periods of aerosol generation other than the scans. Since only a fairly narrow range of frequencies is required in order to determine the resonant frequency, the scan may be a short scan wherein the plurality of frequencies may comprise from about 1, 2 or 5 kHz below the resonant frequency to about 1, 2 or 5 kHz above the resonant frequency, for example from 85 about kHz to about 90 kHz. Since the scan only uses frequencies close to the resonant frequency, the reduction in aerosol output rate can be fully compensated by increasing the duty cycle.

The controller may additionally or alternatively be configured to determine whether liquid is present in the reservoir from the scan. The controller may cease to drive the vibrator if the controller determines that no liquid is present. The scan may be a long scan wherein the plurality of frequencies may comprise from about 10 or 15 kHz below the resonant frequency to about 10 or 15 kHz above the resonant frequency, for example from 75 kHz to about 100 kHz, for optimal determination of whether liquid is present in the reservoir.

The inhalation device may be breath-actuated, i.e. the aerosol is not generated continuously, but only when the patient inhales.

When the controller is configured to perform both short and long scans, the short scans may be performed more frequently than the long scans. For example, the short scans may be performed once or twice per second, and the long scans may be performed once every three, five or ten seconds, or if the nebulized is breath-actuated, once per breath. Having fewer long scans mitigates against the decrease in aerosol output rate, since only these scans involve the frequencies further from resonance at which a 100% duty cycle may not be able to compensate for the reduction in aerosol output rate.

The controller may be configured to additionally perform one or more scans at a constant duty cycle. In particular, a constant duty cycle scan may be performed after it has been determined that no liquid is present in the reservoir on the basis of a scan in which the duty cycle is adjusted, in particular a long scan. The additional scan at constant duty cycle provides confirmation that no liquid is present in the reservoir, but does not result in a decrease in aerosol output rate because it is only performed after the liquid has been aerosolized.

In a second aspect, the invention provides a method of operating an inhalation device of the first aspect of the invention, and/or of operating an inhalation device comprising an aerosol generator having a vibrator and a membrane, and a reservoir for liquid to be aerosolized which is in fluid communication with the membrane, the methods comprising:

a) providing a driver signal comprising a frequency and a duty cycle to operate the vibrator so that the membrane vibrates and generates an aerosol;
b) performing scans in which the membrane is vibrated at a plurality of frequencies;
c) measuring an electrical parameter of the aerosol generator as a function of frequency during the scan;
d) adjusting the duty cycle during the scan in response to the measured value of the electrical parameter in order to minimize a decrease in the value of the electrical parameter.

The method may further comprise determining a resonant frequency of the aerosol generator from the scan. The scan may be a short scan wherein the plurality of frequencies comprises from about 1, 2 or 5 kHz below the resonant frequency to about 1, 2 or 5 kHz above the resonant frequency.

The method may additionally or alternatively comprise determining whether liquid is present in the reservoir from the scan. The scan may be a long scan wherein the plurality of frequencies comprises from about 10 or 15 kHz below the resonant frequency to about 10 or 15 kHz above the resonant frequency. The method may further comprise performing one or more scans at a constant duty cycle once it has been determined that no liquid is present in the reservoir.

The method may comprise performing short scans and performing long scans. The short scans may be performed more frequently than the long scans.

**Brief Description of the Figures**

The invention will now be further described with reference to the Figures, wherein:

Figure 1 shows an expanded view of a vibrating membrane nebulizer.
Figure 2 shows the aerosol generator for the nebulizer of Figure 1.
Figure 3 shows a schematic diagram of the control system for the aerosol generator.
Figures 4A and 4B show spectra obtained without and with liquid in the reservoir respectively.
Figure 5 shows a measure of the aerosol output rate as a function of time, including a scan with a constant duty cycle.

Figure 6 shows a plot of the duty cycle as a function of frequency using the method of the invention.

**Detailed Description of the Invention**

The term "scan" refers to the process of sequentially vibrating the vibrator at a large number of different frequencies in stepwise increments across a defined range, and measuring the value of an electrical parameter at some or all of the frequencies. The term "spectrum" refers to a graph which is obtained by plotting the measured values of the electrical parameter as a function of frequency. The electrical parameter may be the current, voltage, power, impedance and/or the current/voltage phase shift. In particular, the electrical parameter may be the current consumption of the vibrator, or of a power converter which provides the power to the vibrator, or the voltage drop at the vibrator. These parameters can be measured by using one or more current and/or voltage sensors, in a direct or an indirect manner.

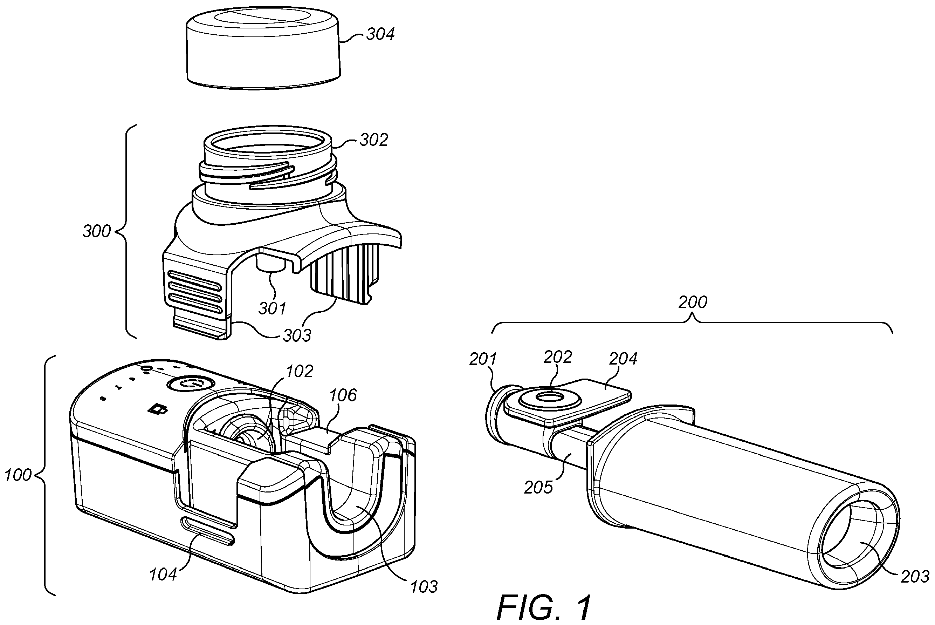

Figure 1 shows an expanded view of a vibrating membrane nebulizer device, which is described in EP2724741 and WO2013/098334. The device comprises three parts: a base unit, a mouthpiece component, and an aerosol head. The base unit **100** has one or more air inlet opening(s) in its rear end (not visible in Figure 1), an air outlet opening **102**, a groove **103** for receiving the mouthpiece component **200**, and one or more key lock members **104**. A channel within the base unit (not visible in Figure 1) connects the air inlet opening(s) to the air outlet opening **102**. The mouthpiece component **200** has an air inlet opening **201** which is attachable to the air outlet opening **102** of the base unit **100**, a lateral opening **202** for receiving an aerosol generator **301**, and an aerosol outlet opening **203**. A channel **205** extends from the air inlet opening **201** to the aerosol outlet opening **203**. The mouthpiece **200** is insertable into the groove **103** of the base unit **100**. The aerosol head **300** comprises the aerosol generator **301**, a filling chamber **302** for the liquid drug formulation to be aerosolized, which is in fluid contact with the upper end of the aerosol generator **301**, and one or more key lock members **303** complementary to the key lock members **104** of the base unit **100**. A lid **304** closes the filling chamber **302** and prevents contamination or spillage of the liquid during use.

The base unit **100**, the mouthpiece **200** and the aerosol head **300** are detachably connectible with one another. The device is assembled by inserting the mouthpiece **200** into the groove **103** in the base unit **100**, then placing the aerosol head **300** over the mouthpiece **200** and engaging the key lock member(s) **303** of the aerosol head **300** with the key lock member(s) **104** of the base unit **100** by gentle pressure on both the aerosol head and the base unit. The aerosol generator **301** is positioned in the aerosol head **300** in such a way that when engaging the key lock member(s), the aerosol generator **301** is inserted into the lateral opening **202** of the mouthpiece **200**. This creates airtight connections between the aerosol generator **301** and the lateral opening **202** in the mouthpiece as well as between the air outlet opening **102** of the base unit **100** and the air inlet opening **201** of the mouthpiece **200**. The base unit **100**, the mouthpiece **200** and the aerosol head **300** can be separated by reversing these steps.

The base unit **100** has one or more indentation(s) **106** positioned at or near the groove **103**, and the mouthpiece **200** has one or more positioning member(s) **204**. The indentation(s) of the base unit are complementary to (i.e. shaped to receive) the positioning member(s) of the mouthpiece. In this context, an indentation is a depression whose "negative" shape is complementary to the "positive" shape of a positioning member, such as a flange, projection or the like. Together, the indentation(s) and positioning member(s) act to position the mouthpiece correctly in the base unit. The indentation(s) and the positioning member(s) may be asymmetrical, so that the mouthpiece can only be inserted into the base unit in one way. This ensures that the device is assembled in such a manner that the position and orientation of the mouthpiece and base unit relative to each other are correct. The base unit contains a controller, such as a printed circuit board (PCB) which controls the operation of the nebulizer.

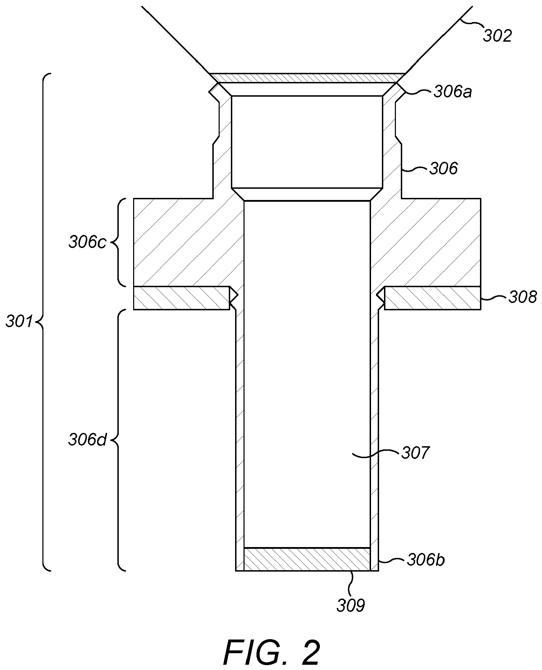

Figure 2 shows the aerosol generator, which is described in detail in WO2008/058941. It comprises a vibrator, e.g. a piezoelectric element **308**, a transducer body **306** and a membrane **309**. The piezoelectric element is preferably an annular single or multi-layer ceramic, which vibrates the transducer body in a longitudinal mode. The transducer body is, for example made of stainless steel, titanium or aluminium, and encloses a cavity **307** which contains liquid to be aerosolized. The inside of the filling chamber **302** is conical so that liquid flows under gravity into the upstream end **306a** of the transducer body and down into the cavity. Together, the filling chamber **302** and cavity **307** form a reservoir for the liquid.

The membrane **309** is positioned at the downstream end **306b** of the transducer body **306**. The holes in the membrane may be formed by electroforming or by laser drilling, with openings normally in the range from about 1 $\mu$m to about 10 $\mu$m. Without vibration of the membrane, the balance of pressures, the shape of the holes and the nature of the material used for the membrane are such that the liquid does not seep out through the membrane. However, vibration of the membrane leads to the formation and emission of aerosol droplets through the holes. The membrane may be made of plastic, silicon, ceramic or more preferably metal, and may be affixed onto or into the downstream end of transducer body by various means, such as gluing, brazing, crimping or laser welding. Optionally, the membrane at least partially forms a dome in its central region, which causes the jet of nascent aerosol droplets to diverge and hence reduces the risk of droplet coalescence.

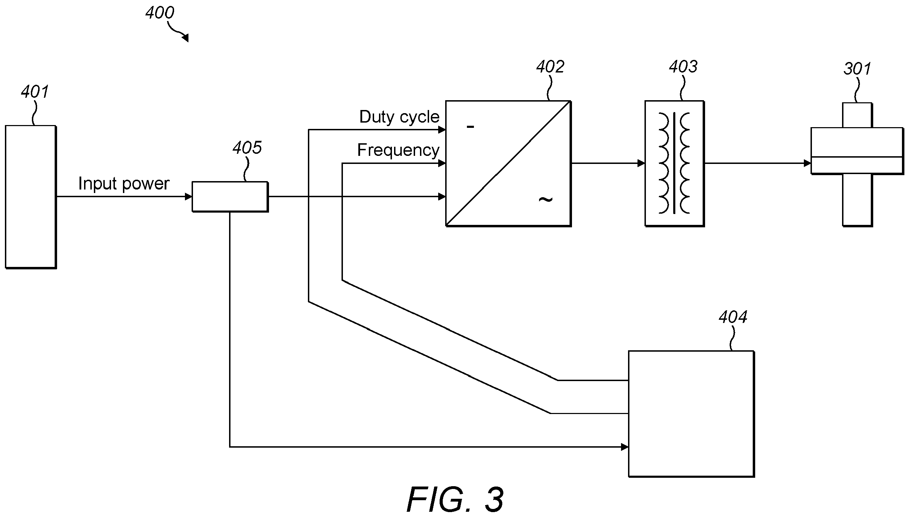

A driver circuit **400**, shown schematically in Figure 3, generates the driver signal that excites the piezoelectric element and hence causes the membrane to vibrate, typically at a frequency in the range of 50 - 200 kHz. The input dc power is provided by a battery **401**. This is converted into an ac driving voltage by a power converter **402** and a transformer **403**. A closed-loop controller **404** controls the power supplied to the aerosol generator **301** by pulse width modulation, by varying the duty cycle, i.e. the fraction of time for which the power is supplied to the aerosol generator. The duty cycle can vary from 0 to 100%. The controller **404** inputs the driving frequency and the duty cycle to the power converter **402**. The controller **404** also measures the current drain of the power converter, by means of a shunt resistor **405** in series with the input of the power converter **402**. The current drain of the power converter is a function of the duty cycle (i.e. the applied stimulus) and the driver frequency. The effective power consumption and the absolute value of the impedance can be derived from the measured current. The aerosol generator is driven using near-resonance driving in which the frequency of the driver signal is as a fixed offset (such as 500Hz or 1 kHz) from the resonant frequency of the aerosol generator (typically around 85kHz).

Excitation of the piezoelectric element causes micronic longitudinal displacements and / or deformations in a direction parallel to the symmetry axis of the transducer body **306**. The transducer body **306** has a region close to the piezoelectric element **308** with a relatively large wall thickness, which serves as a stress concentration zone **306c,** and a region downstream thereof **306d** with a relatively low wall thickness which serves as a deformation amplification zone. This configuration amplifies the vibrations or deformations of the transducer body **306** caused by the piezoelectric element **308**. The piezoelectric element **308** is located at the level of, or adjacent to, the stress concentration zone **306c**. The internal diameter of the transducer body at the deformation amplification zone may be the same as at the stress concentration zone, so that the differences in wall thickness correspond to different external diameters. Alternatively, the external diameter of the transducer body may be constant, while the inner diameters differ at the position of the two zones.

The nebulizer is breath-actuated so that it only generates aerosol when the patient is inhaling. This avoids wasting the aerosol that is generated when the patient is exhaling, as can occur in nebulizers that operate in a continuous manner. A pressure sensor (e.g. a barometric pressure sensor) is located adjacent to, and in pneumatic connection with, the channel in the base unit between the air inlet opening(s) and the air outlet opening **102**. The pressure sensor measures the pressure in the channel, and sends a signal representing the pressure to the controller. When the patient begins to inhale on the mouthpiece, the pressure in the channel drops. If the pressure drops below a certain value, the controller determines that the patient has begun to inhale, and causes the piezoelectric element, and hence the membrane to vibrate, so that aerosol droplets are generated.

When the nebulizer is operated, aerosol is generated by the membrane **309** and released into the channel **205**. Air enters through the air inlets in the base unit and passes through the channel in the base unit, the air outlet opening **102** and the air inlet opening **201** of the mouthpiece component, and into the channel **205** where mixes it with the aerosol. The air and aerosol then flow along the channel **205,** out through the aerosol outlet opening **203** of the mouthpiece and into the patient's airway.

The controller stops the aerosol generation when a pre-set length of time (for example 3s) has elapsed since the aerosol generation started. The pre-set length of time may correspond to the length of a typical inhalation, and may be configurable by the patient. Alternatively, the pre-set length of time may be shorter than a typical inhalation, so that in the final part of the inhalation, the patient receives air but no aerosol. This ensures that the aerosol reaches the central and lower parts of the patient's airway, but is not delivered to the patient's upper airway (e.g. the throat) where it would be ineffective. However, the controller could alternatively detect when the patient ceases to inhale, by sensing the increase in pressure in the channel, and then stop aerosol generation.

The resonant frequency of the aerosol generator changes over the duration of a treatment as the amount of liquid in the reservoir decreases. In order to maintain a fixed offset between the driver signal frequency and the resonant frequency, it is necessary to measure the resonant frequency at intervals throughout operation of the aerosol generator, for example every 0.5s. This is done by scanning the frequency of the driver signal across a range of frequencies from below the resonant frequency to above it, for example from about 1, 2, 5, 10 or 15 kHz below the resonant frequency to about 1, 2, 5, 10 or 15 kHz above the resonant frequency, such as from 75 kHz to about 100 kHz in steps of 0.1kHz, with a constant duty cycle (i.e. constant stimulus). The central frequency for the scan may be the most recently measured value of the resonant frequency, or a pre-set frequency (since the resonant frequency changes slowly within a known range). The current drain of the power converter is sampled at each frequency of the scan.

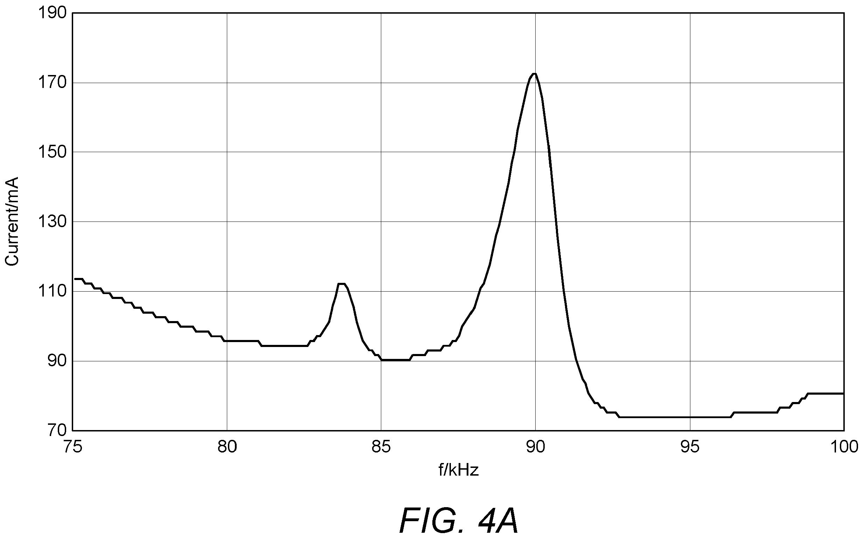

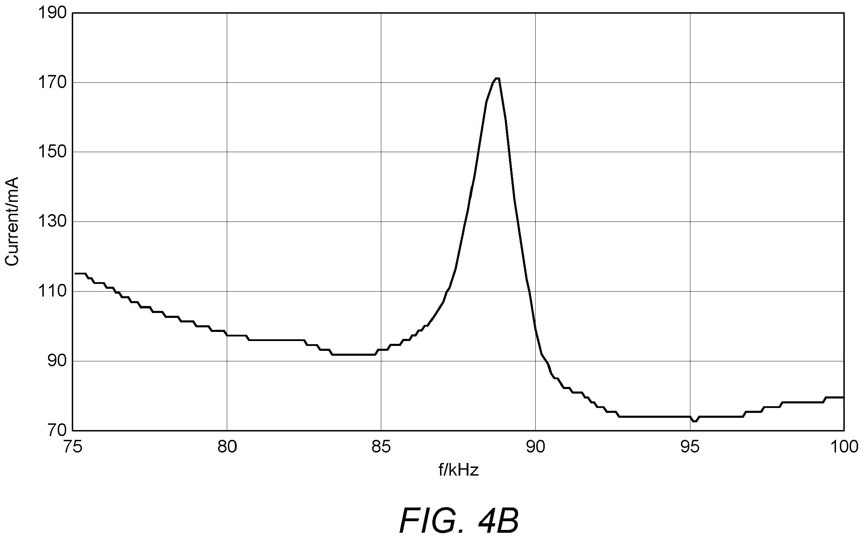

Figures 4A and 4B show the spectra, i.e. the graphs of current as a function of frequency, obtained with the nebulizer of Figure 1, by vibrating the membrane at a series of different frequencies from 75 kHz to 100 kHz in steps of 0.1khZ with a constant duty cycle, and measuring the current drain of the power converter at each frequency.

Figure 4A shows the spectrum when no liquid was present The power consumed by an oscillating system is a maximum at the resonant frequency. The main peak at 90kHz, at which the maximum current consumption occurs, is the resonant frequency of the aerosol generator. The peak has a full width at half maximum of about 2 kHz. There is also a smaller peak at about 84 kHz, which is the resonant frequency of the membrane.

Figure 4B shows the spectrum obtained when liquid was present. Two differences are apparent: the main resonance peak is at a slightly lower frequency, 89 kHz, and the membrane peak has disappeared. The changes in the spectra, such as the shape of the main resonance peak and / or the membrane peak, as the amount of liquid in the reservoir decreases over the course of a number of inhalations may form the basis for determining when the liquid is used up and the reservoir becomes empty.

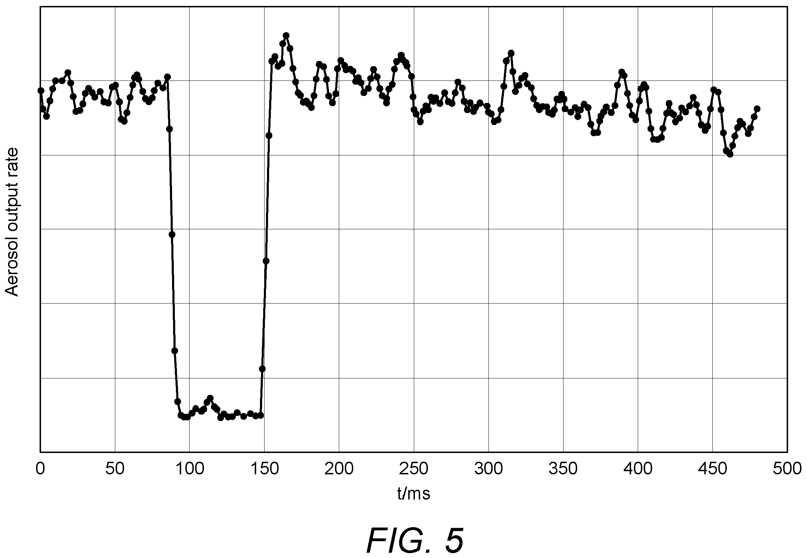

Figure 5 is a graph showing a measure of the aerosol output as function of time over a period of 0.5s. A scan is performed from about t = 85ms to 155ms. During the scan, the aerosol output rate drops almost to zero. This is because most of the scan frequencies are not the optimum driving frequency, so little aerosol is generated. Thus, performing a scan has the disadvantage of interrupting the aerosolization process for approximately 70ms in each period of 500ms. This decreases the average aerosol output rate by about 15%.

The present invention addresses this problem by varying the duty cycle during the scan in order to maintain, as far as is possible, a constant input current to the aerosol generator. In other words, instead of applying a constant duty cycle (stimulus) and measuring the resulting current, the controller reacts to each change in frequency during the scan, and sets the duty cycle as required in order to maintain a constant current, or at least to minimize the drop in the current. (In an analogue system, the same effect could be achieved by increasing the amplitude of the voltage). The target current is pre-set and is chosen in order to achieve the desired aerosol output rate. The controller compares the actual (measured) current drain of the power converter with the target current. The controller adjusts the duty cycle (dc) via a feedback loop to match the actual current ($I_{act}$) to the set target current ($I_{set}$), using a driver coefficient K. The adjustment takes place intermittently, at intervals of 150 μs.

$$dc_{new} = dc_{old} + K\left(I_{set} - I_{act}\right)$$

During normal aerosol generation, the nebulizer typically operates with a target duty cycle of about 50%, with a range from about 30% to 70%, i.e. less than the maximum possible duty cycle of 100%. Thus, by increasing the duty cycle, the power input to the aerosol generator during a scan can be increased by up to a factor of two or three. Consequently, the effective power of the aerosol generator can be maintained, and hence a constant aerosol output rate, at least within a certain range of the resonant frequency. Provided that the frequency is not too far from the resonant frequency, the drop in the current to the aerosol generator can be compensated by increasing the duty cycle. The extent of this frequency range depends on the normal duty cycle: so, for example if the normal duty cycle is 50%, it can be increased by up to a factor of 2, whereas if the normal duty cycle is 33% it can be increased by up to a factor of 3. Typically, a constant aerosol output rate can be maintained over a frequency range of at least about +/- 2 kHz from the resonant frequency. Outside this range, it may not be possible to maintain the constant aerosol output rate; nonetheless, by increasing the duty cycle to its maximum value, the reduction in the aerosol output rate can be substantially reduced. Thus "to minimize a decrease in the value of the electrical parameter" means to maintain a constant value by increasing the duty cycle at frequencies where this is possible, and, to apply the maximum duty cycle (i.e. 100%) at other frequencies so that the value of the electrical parameter is reduced as little as possible.

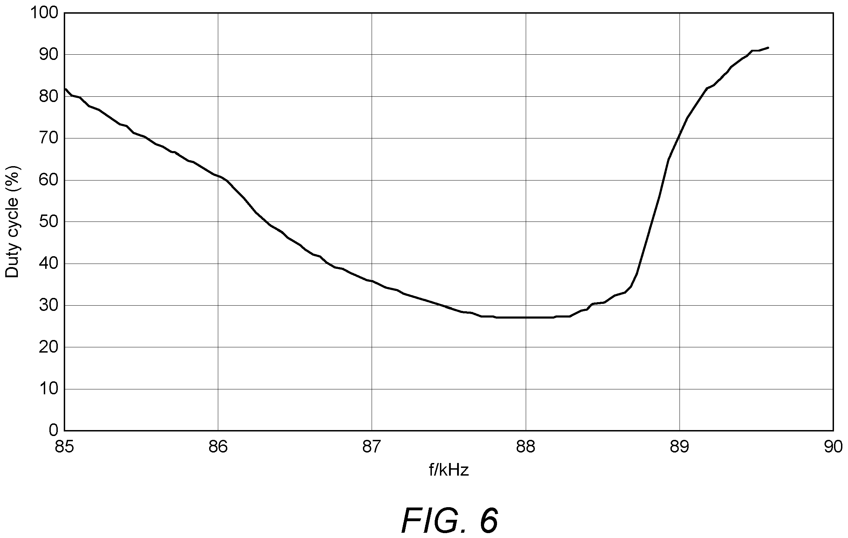

Figure 6 shows the measured spectrum of duty cycle as a function of frequency (around the resonant frequency) with a constant input current to the aerosol generator, with liquid present in the reservoir. A scan to determine the resonant frequency only needs to cover a fairly small range around the resonant frequency, e.g. 1 or 2 kHz above and below it. This curve contains the same information as in Figure 4B (the known method) because the current, duty cycle and frequency form an invariant (for a given liquid level). In this case, the resonant frequency is the frequency at which the duty cycle applied by the controller is smallest. Thus the resonant frequency corresponds to the minimum in the graph of duty cycle as a function of frequency, in this case at 88khz. (It differs slightly from the value of 89 kHz in Fig 4B because different aerosol generators were used for these measurements.)

As with the known (constant stimulus) method, shape of the curve can be used to detect whether liquid is present on the membrane or not. A scan for empty detection typically requires a larger frequency range than that used in Figure 6, such as from about 10 or 15 kHz below the resonant frequency to about 10 or 15 kHz above it. This is because empty detection may rely in whole or in part on features in the spectrum other than the resonance peak, for example the appearance of the membrane peak. Thus, if the nebulizer performs scans for both of these purposes, it is possible to run frequent, short scans to determine the resonant frequency, for example every 0.5s, during which the aerosol output rate can be maintained, and occasional long scans, for example once per inhalation, towards the end of the breath for empty detection. This further mitigates against the drop in aerosol output rate since only the occasional, long scans involve the frequencies further from resonance at which a 100% duty cycle may not be able to compensate for the drop in current consumption.

Furthermore, it is possible to perform scans with a variable duty cycle during the treatment, and then, once the scan indicates that there is no liquid present on the membrane, to perform a scan is with a constant stimulus (as in the known method) to provide independent confirmation that the reservoir is empty. This minimizes the reduction in aerosol output rate during the treatment; the constant duty cycle scan can be used without decreasing the aerosol output because it is only performed after the liquid has been aerosolized. If the controller determines, on the basis of the constant duty cycle scan, that there is in fact liquid present, it may re-run the variable duty cycle scan and / or the constant duty cycle scan until the determination of whether or not liquid is present is the same from both types of scan.

The invention can be used in many vibrating membrane nebulizers, for example of the types described in US 9 027 548, WO 2012 /046220 and WO 2015 / 193432. In these, the membrane is mounted directly on the piezoelectric element, or there is an annular, planar support member on which the membrane and/or the piezoelectric element are mounted. However, the invention is especially advantageous in nebulizers of the type described in EP2724741 and WO2013/098334. In this type of nebulizer, the resonant frequency depends quite strongly on the liquid fill level in the reservoir due to the tubular transducer body. Consequently, it is necessary to frequently re-adjust the driver frequency. Since the scan has to be performed many times during nebulization, the effect of the reduced aerosol output during the scan on the total aerosol output rate is larger than in nebulizers where the scan is performed less frequently.

## Claims

1. An inhalation device comprising:

    • an aerosol generator comprising a vibrator and a membrane,
    • a reservoir for liquid to be aerosolized which is fluidically connected to the membrane,
    • a controller which is configured (i) to provide a driver signal to operate the vibrator so that the membrane vibrates and generates an aerosol, the driver signal comprising a frequency and a duty cycle; (ii) to perform scans in which the membrane is vibrated at a plurality of frequencies; and (iii) to measure an electrical parameter of the aerosol generator as a function of frequency during the scan;

    **characterized in that** the controller is a closed-loop controller which is configured to adjust the duty cycle during the scan in response to the measured value of the electrical parameter in order to minimize a decrease in the value of the electrical parameter.

2. An inhalation device according to claim 1, which comprises a battery, a transformer and a power converter, wherein the controller provides the driver signal to the power converter.

3. An inhalation device according to claim 2 wherein the electrical parameter is the current drain of the power converter.

4. An inhalation device according to any of claims 1 to 3 wherein the aerosol generator has a support member comprising a hollow tubular body having a flange at or close to a first end onto which the vibrator is attached, and a second end into or onto which the membrane is mounted.

5. An inhalation device according to any of claims 1 to 4, wherein the controller is configured to determine a resonant frequency of the aerosol generator from the scan.

6. An inhalation device according to claim 5, wherein the plurality of frequencies comprises from about 1, 2 or 5 kHz below the resonant frequency to about 1, 2 or 5 kHz above the resonant frequency.

7. An inhalation device according to any of claims 1 to 6, wherein the controller is configured to determine whether liquid is present in the reservoir from the scan.

8. An inhalation device according to claim 7, wherein the plurality of frequencies comprises from about 10 or 15 kHz below the resonant frequency to about 10 or 15 kHz above the resonant frequency.

9. A method of operating an inhalation device comprising an aerosol generator comprising a vibrator and a membrane, and a reservoir for liquid to be aerosolized which is in fluid communication with the membrane, the method comprising:

    a) providing a driver signal comprising a frequency and a duty cycle to operate the vibrator so that the membrane vibrates and generates an aerosol;
    b) performing scans in which the membrane is vibrated at a plurality of frequencies;
    c) measuring an electrical parameter of the aerosol generator as a function of frequency during the scan;
    d) adjusting the duty cycle during the scan in response to the measured value of the electrical parameter in order to minimize a decrease in the value of the electrical parameter.

10. A method of operating an inhalation device according to claim 9, further comprising determining a resonant frequency of the aerosol generator from the scan.

**11.** A method of operating an inhalation device according to claim 10, wherein the scan is a short scan in which the plurality of frequencies comprises from about 1, 2 or 5 kHz below the resonant frequency to about 1, 2 or 5 kHz above the resonant frequency.

**12.** A method of operating an inhalation device according to any of claims 9 to 11 further comprising determining whether liquid is present in the reservoir from the scan.

**13.** A method of operating an inhalation device according to claim 12, wherein the scan is a long scan in which the plurality of frequencies comprises from about 10 or 15 kHz below the resonant frequency to about 10 or 15 kHz above the resonant frequency.

**14.** A method of operating an inhalation device according to claim 11 and claim 13 wherein the controller is configured to perform short scans more frequently than long scans.

**15.** A method of operating an inhalation device according to any of claims 12 to 14, further comprising performing one or more scans at a constant duty cycle after it has been determined that no liquid is present in the reservoir.

FIG. 1

FIG. 2

FIG. 3

EP 4 015 094 A1

EP 4 015 094 A1

*FIG. 4A*

EP 4 015 094 A1

*FIG. 4B*

*FIG. 5*

EP 4 015 094 A1

*FIG. 6*

Europäisches
Patentamt

European
Patent Office

Office européen
des brevets

# EUROPEAN SEARCH REPORT

Application Number

EP 20 21 4450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/129813 A1 (LITHERLAND CRAIG [US] ET AL) 19 September 2002 (2002-09-19) * paragraphs [0001], [0041] - [0013], [0055]; figures 1-5 * | 1-15 | INV. B05B17/06 A61M15/00 B05B17/00 |
| Y | US 2020/156098 A1 (GREHAN JOSEPH [IE] ET AL) 21 May 2020 (2020-05-21) * the whole document * | 1-13,15 | |
| Y | US 2003/164658 A1 (SARAF SHAILENDHAR [US]) 4 September 2003 (2003-09-04) * the whole document * | 1-13,15 | |
| A | US 2009/026883 A1 (CROFT NATHAN JAMES [GB] ET AL) 29 January 2009 (2009-01-29) * paragraphs [0003], [0008]; figures 1-6 * | 1 | |
| A | US 2016/361506 A1 (LIN JUNG-YU [TW] ET AL) 15 December 2016 (2016-12-15) * paragraph [0053]; figures 1-8 * | 1 | |

| | TECHNICAL FIELDS SEARCHED (IPC) |
|---|---|
| | B05B A61M |

The present search report has been drawn up for all claims

| Place of search | Date of completion of the search | Examiner |
|---|---|---|
| Munich | 31 May 2021 | Verger, Paul |

EPO FORM 1503 03.82 (P04C01)

## ANNEX TO THE EUROPEAN SEARCH REPORT
## ON EUROPEAN PATENT APPLICATION NO.

EP 20 21 4450

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2021

| Patent document cited in search report | | Publication date | Patent family member(s) | | Publication date |
|---|---|---|---|---|---|
| US 2002129813 | A1 | 19-09-2002 | CA | 2441190 A1 | 10-10-2002 |
| | | | EP | 1370129 A1 | 17-12-2003 |
| | | | US | 2002129813 A1 | 19-09-2002 |
| | | | WO | 02078424 A1 | 10-10-2002 |
| US 2020156098 | A1 | 21-05-2020 | EP | 3672734 A1 | 01-07-2020 |
| | | | US | 2020156098 A1 | 21-05-2020 |
| | | | WO | 2019038408 A1 | 28-02-2019 |
| US 2003164658 | A1 | 04-09-2003 | AU | 2003213608 A1 | 22-09-2003 |
| | | | US | 2003164658 A1 | 04-09-2003 |
| | | | WO | 03077055 A2 | 18-09-2003 |
| US 2009026883 | A1 | 29-01-2009 | CN | 101378846 A | 04-03-2009 |
| | | | EP | 1981651 A1 | 22-10-2008 |
| | | | GB | 2435133 A | 15-08-2007 |
| | | | GB | 2435136 A | 15-08-2007 |
| | | | JP | 2009525860 A | 16-07-2009 |
| | | | US | 2009026883 A1 | 29-01-2009 |
| | | | WO | 2007091027 A1 | 16-08-2007 |
| US 2016361506 | A1 | 15-12-2016 | EP | 3103496 A1 | 14-12-2016 |
| | | | US | 2016361506 A1 | 15-12-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014062175 A
- WO 2015091356 A
- EP 2724741 A
- WO 2013098334 A
- WO 2008058941 A
- US 9027548 B
- WO 2012046220 A
- WO 2015193432 A